# EUROPEAN PATENT APPLICATION

(11) **EP 2 508 534 A1**
(43) Date of publication of application: **10.10.2012**
(21) Application number: 10834424.3
(22) Date of filing: 08.09.2010
(51) Int. Cl.: C07K 14/47, A23L 1/22, C12N 15/09

(54) **HUMAN SWEET TASTE RECEPTOR AGONIST SWEETNESS REGULATING SUBSTANCE FOR SWEET SUBSTANCES**

(30) Priority: 02.12.2009 JP 2009274977
(71) Applicant: T. Hasegawa Co., Ltd., Tokyo 103-8431 (JP); The University of Tokyo, Bunkyo-Ku Tokyo 113-8654 (JP)
(72) Inventor: ABE, Keiko, Tokyo 113-8654 (JP); MISAKA, Takumi, Tokyo 113-8654 (JP); FUJIWARA, Satoshi, Kawasaki-shi Kanagawa 211-0022 (JP)
(74) Representative: Marek, Pierre
(86) International application number: PCT/JP2010/065367
(87) International publication number: WO 2011/067971

(57) **Abstract**

[Object] An object of the present invention is to provide a human sweet taste receptor-acting sweet taste regulating substance to a sweet taste substance by which the advantageous effects such as improvement in a taste, saving of a sweetener, reduction in calorie, low caries etc. can be obtained by applying to various foods and beverages.

[Solution] The sweet taste receptor-acting sweet taste regulating substance of the present invention is identified by measurement of a physiological response by a sweet taste substance, using a cultured cell strain which is allowed to express hT1R2 and hT1R3, and a G protein α subunit, by transferring an expression construct obtained by inserting respective cDNAs encoding the hT1 R2 and the hT1 R3, and the G protein α subunit into the same plasmid, into a 293 cell in which an FRT (Flippase Recognition Target) site has been incorporated into one place in a genome DNA.

## Description

### Technical Field

The present invention relates to a human sweet taste receptor-acting sweet taste regulating substance to a sweet taste substance, more particularly, to a human sweet taste receptor-acting sweet taste regulating substance to a sweet taste substance, obtained by objectively assessing a sweet taste intensity, using a cultured cell strain which is allowed to functionally stably express both of a human sweet taste receptor (hT1R2+hT1R3) and a G protein α subunit.

### Background Art

Taste sense is the sense generated by binding of a specific receptor present, particularly, on a surface of a tongue with a substance, when a substance is placed in a mouth. The taste sense of a mammal is constructed of five fundamental tastes, that is, a salty taste, a sour taste, a sweet taste, an umami taste and a bitter taste, and is considered to be formed by integration of these fundamental tastes. Currently, it is said that a salty taste and a sour taste are sensed via some ion channel-type receptors expressed on a cell membrane on a proximal side of taste cells present in taste buds on a surface of a tongue and, it is considered that an ion channel-type receptor consisting of PKD2L1+PKD1L3, which are a TRP channel family, functions, particularly, regarding a sour taste.

On the other hand, regarding a sweet taste, an umami taste and a bitter taste, it is considered that those tastes are sensed by signal transduction via a G protein coupled receptor (GPCR) which is a membrane protein present in taste cells, and a G protein coupling with it. Specifically, it has been revealed that a sweet taste is received with a heterodimer of T1R2+T1R3 (sweet taste receptor), an umami taste is received with a heterodimer of T1R1 +T1 R3 (umami taste receptor), and a bitter taste is received with about 30 kinds of molecules named as T2R family (bitter taste receptor).

The G protein is constructed of three subunits of α, β and γ. The state where α, β and γ subunits are connected is an inactive type and, when a taste substance binds to a G protein coupled receptor, GDP (guanosine 5' diphosphate) which has been bound to an α subunit is substituted with GDP, resulting in an active type in which the G protein has been dissociated into a binding body of a GTP-α subunit and a β-γ subunit.

The construction of a transduction mechanism of taste sense information has not been completely clarified, but is generally understood as follows. That is, a popular opinion is that, first, when a taste substance is bound with a receptor of taste cells, a calcium concentration in cells is raised via an information transduction process through a second messenger (IP₃, DAG) and the like in cells. A calcium ion supplied into cells then releases a neurotransmitter into a synapse to generate an action potential in nerve cells and, as a result, a taste sense signal starting from the receptor is transduced from a taste nerve to a brain, and the taste sense information is discriminated and determined. Recently, a theory is also being accepted, that a calcium ion opens a novel cation channel called TRPM5, and an inflow of a monovalent cation into cells causes depolarization.

Among the above-mentioned five fundamental tastes consisting of a salty taste, a sour taste, a sweet taste, an umami taste and a bitter taste, particularly, a sweet taste is a taste which is felt very tasty when a sugar content in blood is decreased. Since a sweet taste gives an image as an energy source including a sugar to a human and, further, causes a stronger satisfactory feeling and a stronger happiness feeling as compared with other fundamental tastes, and is deeply involved in emotion of a human, it is a taste central to determination of preference of beverages and foods.

However, a sweet taste has a higher minimum sensitivity (threshold) as compared with other fundamental tastes, and has a property that a sweet taste is sensed with difficulty in a small amount. On the other hand, when a sweet taste is too strong, this results in inducing an unhealthy image such as high calorie and obesity. For this reason, when beverages and foods with a sweet taste imparted thereto are produced, it is important to regulate an intensity of a sweet taste so that the sweet taste can be sensed by a human, and preferably accepted.

In order to improve a savor of foods and beverages or the like, a food flavor has previously been used. A savor is a perceptual-psychological feeling which is sensed from a taste sense, a smell sense or the like. A flavor is also used when an intensity of a sweet taste is enhanced, and as a flavor whose sweet taste enhancing effect has actually been confirmed, there are, for example, ethyl butyrate, ethyl vanillin, sedanenolide, alapyridaine and the like.

Generally, the savor improving effect due to a flavor is thought to be action through a smell sense by a fragrance possessed by the flavor. The savor improving effect of the flavor has previously been verified mainly by sensory evaluation of a human. However, since in the sensory evaluation, information sensed from a taste sense and a smell sense is integratively assessed, it is difficult to verify whether or not the flavor acts on a taste sense to have the savor improving effect. Particularly, it is very difficult to objectively assess a difference in an extent of improvement in savor through a taste sense, between a plurality of flavors. In the sensory evaluation or sensory test, a variation of evaluation between well trained assessors (called panel or panelist) cannot be neglected.

It is also considered to isolate taste cells present in taste buds on a surface of a tongue, and using these isolated taste cells to assess a sweet taste intensity, in place of sensory evaluation by a human. However, since taste cells isolated from those present in taste buds on a surface of a tongue are very weak in adhesion onto a culturing plate, and immediately die, they cannot be used in time-consuming assessment.

Since a sweet taste is a taste central to determination of preference of beverage and foods as described above, for example, if a substance acting on a sweet taste receptor to enhance a sweet taste can be identified, the advantageous effects such as improvement in a taste of beverage, foods and medicaments, reduction in a use amount of a sweetener, and reduction in ingested calorie can be attained. Therefore, great interest is given to such a sweet taste enhancing substance from the industrial world of beverages, foods, flavors and the like.

Then, in order to respond to such an interest from the industrial world, as a method of objectively identifying a sweet taste regulating substance, an assay using a cell strain which is allowed to express a sweet taste receptor being a heterodimer of T1R2+T1R3 has been reported.
Specifically, for example, in Example 11 of Patent Literature 1, there has been reported identification of a sweet taste regulating compound using a cell strain produced by co-expressing hT1 R2/hT1 R3 by transfecting a linearized pEAK10 (Edge Biosystems)-derived vector comprising an expression construct of hT1R2 (plasmid SAV2486), and a pCDNA 3.1/ZEO-derived (Invitogen) vector comprising an expression construct of hT1R3 (plasmid SXV550) into a Gα15-expressing cell strain (HEK-293 cell strain of Aurora Bioscience).

And, there is described in Patent Literature 2 that a stable HEK-293 cell expressing a domain named as hT1 R2-1 consisting of an hT1R2 N-terminal extracellular domain and an hT1R1 C-terminal seven times transmembrane domain, hT1R3, and a chimeric G protein G16-t25, and a stable HEK-293 cell stably expressing a domain named as hT1 R1-2 consisting of an hT1R1 extracellular domain and an hT1R2 C-terminal seven times transmembrane domain, hT1R3, and a chimeric G protein G16g44 are useful in an assay for identifying a taste sense altering substance of a sweet taste.

And, in Patent Literature 3, there is described an assay for identifying a sweet taste enhancing substance, using a cell line obtained by transfecting a linearized pcDNA4-T0 vector comprising a cDNA encoding a human receptor protein T1 R2-TMD or a CSR:T1R chimeric receptor into a HEK-293T cell expressing Gα16gust44.

### Citation List

### Patent Literature

Patent Literature 1: JP-A-2008-13570
Patent Literature 2: JP-T-2009-517003
Patent Literature 3: WO 2007/121604

### Summary of Invention

### Technical Problem

All of the previous cell lines expressing a sweet taste receptor are prepared by preparing or obtaining cells expressing a predetermined G protein in advance, and transfecting a vector comprising a gene encoding T1R1, and a vector comprising a gene encoding T1R3 into this, respectively.

However, when a cell line is prepared by such a method, an efficiency of introducing each vector into cells is not the same, wherein some may be preferentially introduced in some cases. Also, there are cases in which an introduced objective gene is randomly inserted into a genome of cells and, in the extreme case, an introduced objective gene is inserted into an inactive part to be not transcribed in a genome.
For this reason, in the previous cell line, a constant amount is expressed, and a difference in its expression amount is slightly recognized between cells with respect to a G protein, but a great variation is recognized between cells with respect to an expression amount and an expression ratio of hT1R2 and hT1R3.

Among the previous cell lines, either one or both of T1R2 and T1R3 are not expressed and, among them, there are cell lines which are not substantially provided with a series of signal transduction mechanisms in cells. Therefore, it could not be said they are functional cell lines.

As described above, the previous cell line expressing a sweet taste receptor does not reflect a sweet taste reception response mechanism of a human, and was not an appropriate model for screening a sweet taste regulating substance acting on a sweet taste receptor.
Therefore, even when a candidate substance of a sweet taste regulating substance is screened using a cell strain expressing a sweet taste receptor which is prepared by the previous method, a selected substance could not be said to be a substance verified to actually act on a human sweet taste receptor to exert the sweet taste regulating effect.

In light of these circumstances, an object of the present invention is to provide a human sweet taste receptor-acting sweet taste regulating substance to a sweet taste substance, whose sweet taste regulating effect through a taste sense has been objectively confirmed, by an assay using a stable cultured cell highly expressing hT1R2 and hT1R3 equally.

### Solution to Problem

The present inventors have intensively studied so as to achieve the above-mentioned object and found that an in vitro experimental system using a stable cultured cell highly expressing hT1R2 and hT1R3 equally, specifically, a cultured cell which is allowed to express hT1R2 and hT1R3, and a G protein α subunit, by gene-transferring an expression construct in which cDNAs encoding the hT1R2 and the hT1R3, and the G protein α subunit are introduced into the same plasmid, into a predetermined cell, is a model which is provided with a reception system close to that of a taste cell feeling a sweet taste, and is very suitable for selecting a sweet taste regulating substance acting on a human sweet taste receptor. Thus, the present invention has been completed based on such a finding.

Thus, the present invention provides a human sweet taste receptor-acting sweet taste regulating substance to a sweet taste substance, which is identified by measurement of a physiological response by a sweet taste substance, using a cultured cell strain which is allowed to express hT1R2 and hT1R3, and a G protein α subunit, by transferring an expression construct obtained by inserting respective cDNAs encoding the hT1R2 and the hT1R3, and the G protein α subunit into the same plasmid, into a 293 cell in which an FRT (Flippase Recognition Target) site has been incorporated into one place in a genome DNA.

### Advantageous Effects of Invention

The present invention can provide a human sweet taste receptor-acting sweet taste regulating substance, whose sweet taste regulating effect through a human sweet taste receptor has been objectively confirmed, and the advantageous effects such as improvement in a taste, saving of a sweetener, reduction in calorie, and low caries can be obtained by application to a variety of foods and beverages.

### Brief Description of Drawings

Fig. 1 is a diagram showing structure of pcDNA5/FRT (Invitrogen).
Fig. 2 is a diagram showing a construct of pcDNA5/FRT (Invitrogen) having a sequence in which a cDNA encoding hT1R2 and a cDNA encoding hG16gust44 are connected so that an IRES sequence is flanked by the cDNAs, downstream of an EF-1α promoter, and in which a sequence in which a cDNA encoding hT1R3 and a cDNA encoding hG16gust44 are connected so that an IRES sequence is flanked by the cDNAs, is inserted downstream of a CMV promoter present downstream of this sequence.
Fig. 3 is a sucrose concentration ― response curve in case of administering neohesperidin dihydrochalcone, cyclamate, p-methoxycinnamic aldehyde and cyclotene in each predetermined concentration based on sucrose to cells which are allowed to express hT1R2 and hT1R3, and hG16gust44.
Fig. 4 is a diagram showing fluorescent images in case of administering neohesperidin dihydrochalcone, cyclamate, p-methoxycinnamic aldehyde and cyclotene in each predetermined concentration based on sucrose to cells which are allowed to express hT1R2 and hT1R3, and hG16gust44.
Fig. 5 is an aspartame concentration―response curve in case of administering neohesperidin dihydrochalcone in a predetermined concentration based on aspartame to cells which are allowed to express hT1R2 and hT1R3, and hG16gust44.
Fig. 6 is a stevia concentration―response curve in case of administering neohesperidin dihydrochalcone in a predetermined concentration based on stevia to cells which are allowed to express hT1R2 and hT1R3, and hG16gust44.
Fig. 7 is a saccharin concentration―response curve in case of administering neohesperidin dihydrochalcone in a predetermined concentration based on saccharin to cells which are allowed to express hT1R2 and hT1R3, and hG16gust44.
Fig. 8 is a diagram showing fluorescent images in case of administering ethylvanillin or maltol in each predetermined concentration based on sucrose to cells which are allowed to express hT1R2 and hT1R3, and hG16gust44.

### Description of Embodiments

The present invention will be further explained in detail below.
In the present invention, a human sweet taste receptor-acting sweet taste regulating substance to a sweet substance means a substance which alters a physiological response from a human sweet taste receptor, that is, an intensity of a sweet taste obtained from a certain specified sweet taste substance alone, by acting on a human sweet taste receptor (hT1 R2+hT1 R3), when the regulating substance is present with the sweet taste substance. Specifically, examples include a human sweet taste receptor-acting sweet taste enhancing substance which enhances a physiological response from a human sweet taste receptor by a sweet taste substance to increase an intensity of a sweet taste, a human sweet taste receptor-acting sweet taste inhibiting substance which decreases a physiological response to decrease an intensity of a sweet taste, etc. The human sweet taste receptor-acting sweet taste regulating substance also includes a substance which is a sweet taste substance itself.

There is no particular limitation on a sweet taste substance being a subject for which a sweet taste is regulated using the human sweet taste receptor-acting sweet taste regulating substance of the present invention, as long as it is a substance exhibiting such a sweet taste that can be sensed by a human, and examples thereof widely include saccharide-based sweeteners such as glucose, fructose, galactose, raffinose, xylose, sucrose, maltose, lactose, starch syrup, isomerized sugar, isomaltooligosaccharide, fructooligosaccharide, galactooligosaccharide, xylooligosaccharide, lactooligosaccharide, soybean oligosaccharide, trehalose, sorbitol, mannitol, maltitol, xylitol, erythritol, lactitol, isomaltol, reduced starch syrup, reduced palatinose, Wasanbon (refined Japanese sugar), brown cane sugar, brown soft sugar, honey, molasses, licorice extract, and maple syrup, and non-saccharide-based sweeteners such as aspartame, saccharin, dulcin, stevioside, stevia extract, glycyrrhizin, Acesulfame-K, sucralose (registered trademark: San-Ei Gen F.F.I), cyclamate, alitame, neotame, perillatin, monellin, curculin (registered trademark: ADEKA) and the like.

The human sweet taste receptor is a heterooligomer receptor constructed by combining two subunits of hT1R2 and hT1R3 as described above, and includes all portions of respective subunits, that is, all regions of a transmembrane domain consisting of seven transmembrane regions, and corresponding cytoplasmic and extracellular loops, a Venus Fly Trap domain, a high cysteine domain and a C-terminal domain.

As the human sweet taste receptor-acting sweet taste enhancing substance of the present invention, specifically, neohesperidin dihydrochalcone (NHDC) (Chemical Formula 1), cyclamate (Chemical Formula 2), p-methoxycinnamic aldehyde (Chemical Formula 3), and cyclotene (Chemical Formula 4) are exemplified. Chemical structures of respective substances are shown below.

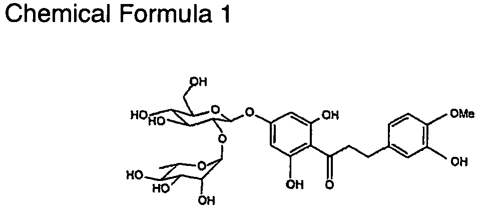

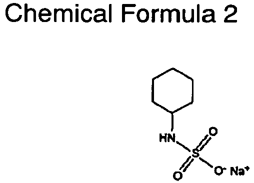

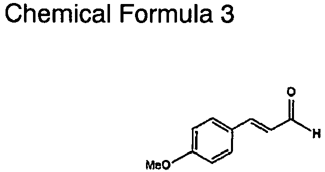

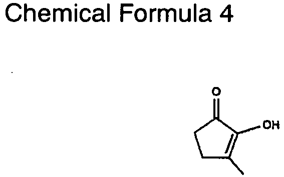

Specific examples of the human sweet taste receptor-acting sweet taste inhibiting substance of the present invention include lactisole (Chemical Formula 5), vanillin (Chemical Formula 6), ethylvanillin (Chemical Formula 7), creosol (Chemical Formula 8), and maltol (Chemical Formula 9). Chemical structures of respective substances are shown below.

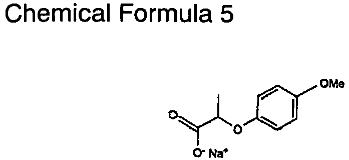

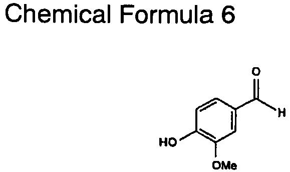

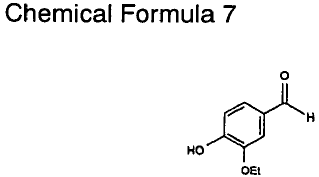

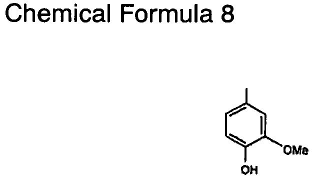

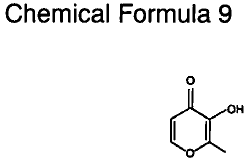

A human sweet taste receptor-acting sweet taste enhancing agent of the present invention is a preparation containing the human sweet taste receptor-acting sweet taste enhancing substance of the present invention as an active ingredient. The enhancing substance may be directly used as it is, or the enhancing substance may be optionally used by dissolving it in a suitable liquid, or mixing with a powder and, if necessary, the enhancing substance is used by appropriately adding an emulsifier, a suspending agent, a stabilizer or the like. The concentration of the active ingredient may be appropriately determined in view of a desired extent of a sweet taste or the like.

A human sweet taste receptor-acting sweet taste suppressing agent of the present invention is a preparation containing the human sweet taste receptor-acting sweet taste suppressing substance of the present invention as an active ingredient. The enhancing substance may be directly used as it is, or the enhancing substance may be optionally used by dissolving it in a suitable liquid, or mixing with a powder and, if necessary, the enhancing substance is used by appropriately adding an emulsifier, a suspending agent, a stabilizer or the like. A concentration of the active ingredient may be appropriately determined in view of a desired extent of a sweet taste or the like.

The human sweet taste receptor-acting sweet taste regulating substance of the present invention was identified by using a cultured cell strain which is allowed to express hT1R2 and hT1R3, and a G protein α subunit, by transferring an expression construct obtained by inserting respective cDNAs encoding the hT1R2 and the hT1R3, and the G protein α subunit into the same plasmid, into a 293 cell in which an FRT (Flippase Recognition Target) site has been incorporated into one place in a genome DNA, by:
(i) contacting a certain specified sweet taste substance with the above-mentioned cultured cell strain, and measuring a generated physiological response,
(ii) contacting a candidate substance of a human sweet taste receptor-acting sweet taste regulating substance which has been added to the sweet taste substance, with the cultured cell strain, and measuring a generated physiological response,
(iii) comparing the measured results of the physiological responses obtained in the steps (i) and (ii), respectively.

Respective cDNAs encoding hT1R2 and hT1R3, and a G protein α subunit may be those obtained by any method, and can be obtained by the known method, for example, a method of cloning cDNAs from mRNAs encoding the proteins, chemical synthesis based on the known nucleotide sequence information, and a method of isolating a genome DNA and splicing it. Other various genes used in the present invention are similarly obtained. A cDNA means a complementary DNA, and is a reverse transcription reaction product of an mRNA transcription product.

Examples of a cDNA encoding a G protein α subunit include a cDNA encoding G15, hG16, or a mutant in which a portion of a sequence thereof is altered. Particularly, when a human sweet taste receptor is allowed to be expressed, a cDNA encoding hG16gust44 is preferable in that information transmission between an intracellular effector can be effectively carried. hG16gust44 is a chimeric G protein in which 44 amino acid residues at a C-terminal portion of human Gα16 are replaced with Ggust.

In the present invention, an expression construct means a nucleic acid molecule for transferring a DNA fragment in which a desired coding sequence, and a suitable nucleic acid sequence such as a promoter, a terminator, a marker gene, and a gene encoding an FRT site necessary for expressing the coding sequence are connected, into a 293 cell in which an FRT site has been incorporated into one place in a genome DNA, and has the same meaning as that of an expression vector.
There is no particular limitation on the promoter functioning as a transcription initiation signal, as long as it has the function of expressing a gene to be introduced, in the 293 cell, and examples thereof include CMV (cytomegalovirus), EF-1α, SRα, CAG and the like. Examples of the terminator which is a nucleic acid sequence terminating transcription include SV40 pA. SV40 pA is contained on a 273 bp BamHI/BcII restriction enzyme fragment, and brings about both of termination and polyadenylation, and many terminators derived from a bovine growth hormone (BGH) are utilized.
And, as a marker gene which is a gene being introduced as a mark for confirming that an objective gene has been introduced, a hygromycin-resistant gene, a puromycin-resistant gene, a neomycin-resistant gene, a kanamycin-resistant gene, a chloramphenicol-resistant gene etc. are exemplified.
And, a sequence of nucleotides 2087 to 2147 disclosed in pFRTβGAL (STRATAGE, protocol, SEQUENCE AND SITES, Catalog#218403, May 28, 1991) corresponds to the gene encoding an FRT site.

There is no particular limitation on the above-mentioned same plasmid into which respective cDNAs are inserted, as long as it can express hT1R2 and hT1R3, and a G protein α subunit in a 293 cell in which an FRT site has been incorporated into one place in a genome DNA. Specific examples thereof include pFRT/lacZeo, pUC12, pUC13, pUC19, pBR322, pBR325, pSH15, pSH19, pUB110, pC194 and the like.

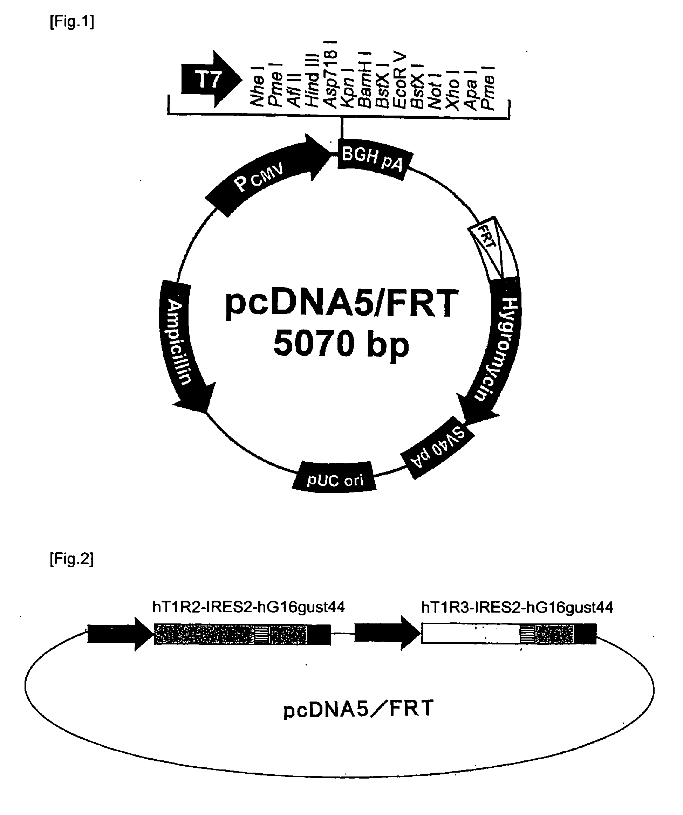

In the present invention, as the plasmid, pcDNA5/FRT (5070 bp, Invitrogen) in which a hygromycin B-resistant gene is arranged on one side of a gene encoding an FRT site is suitably used. pcDNA5/FRT (Invitrogen) has a hygromycin B-resistant gene and, when recombination is generated at an FRT site of a host cell having an FRT site, the host cell becomes hygromycin B-resistant and Zeocin-sensitive. On the other hand, when it is not incorporated into an FRT site, but is incorporated into another site, the host cell becomes hygromycin B-sensitive and, therefore, whether an objective gene has been incorporated into an FRT site or not can be easily discriminated. A structure of pcDNA5/FRT (5070 bp, Invitrogen) is shown in Fig. 1.

As an expression construct obtained by inserting cDNAs encoding hT1R2 and hT1R3, and a G protein α subunit into the same plasmid, pcDNA5/FRT (Invitrogen) having a sequence in which a cDNA encoding hT1R2 and a cDNA encoding hG16gust44 are connected so that an IRES sequence is flanked by the cDNAs, downstream of an EF-1α promoter, and in which a sequence in which a cDNA encoding hT1R3 and a cDNA encoding hG16gust44 are connected so that an IRES sequence is flanked by the cDNAs, is inserted downstream of a CMV promoter present downstream of this sequence, is preferable (Fig. 2). The reason is that, by using the expression construct, hT1R2, hT1R3, and hG16gust44 can be expressed simultaneously, and a cultured cell strain having very good sensitivity to a ligand acting on a sweet taste receptor can be obtained.

The expression construct obtained by inserting cDNAs encoding hT1R2 and hT1R3, and a G protein α subunit into the same plasmid defined in the present invention can be prepared, for example, according to the following steps (a) to (g).
(a) Substitution of 6 bases is performed at places other than a multicloning site (base number 895 to 1010) of pcDNA5/FRT (Invitrogen), and a recognition sequence (5'-GATATC-3') of a restriction enzyme EcoRV is newly prepared. As one preferable aspect of this step (a), an aspect of introducing a recognition sequence of EcoRV into immediately under (base number 18 to 23) a recognition sequence (base number 12 to 17) of a restriction enzyme Bgl II of pcDNA5/FRT (Invitrogen) is exemplified. This aspect can be performed, for example, according to the following substeps (a1) to (a5).
(a1) A sense primer having a recognition sequence (5'-AGATCT-3') of Bgl II at a 5'end, and a recognition sequence of EcoRV immediately under therefrom is designed and prepared. On the other hand, an antisense primer having a sequence in a BGH pA sequence is designed and prepared.
(a2) Using the sense primer and the antisense primer prepared in (a1) and, employing pcDNA5/FRT (Invitrogen) as a template, a polymerase chain reaction (PCR) is performed to amplify a DNA fragment in which respective recognition sequences of Bgl II and EcoRV are connected.
(a3) The DNA fragment amplified in (a2) is digested with Bgl II and Not I.
(a4) pcDNA5/FRT (Invitrogen) is digested with Bgl II and Not I.
(a5) The DNA fragment obtained in (a3) and pcDNA5/FRT (Invitrogen) obtained in (a4) are connected by a ligation reaction to prepare a vector having a recognition sequence of EcoRV immediately under a recognition sequence of Bgl II of pcDNA5/FRT (Invitrogen).
The ligation reaction may be performed by using a usual T4 DNA ligase, but in order to perform treatment rapidly and simply, it is preferable to use Ligation high Ver.2 (TOYOBO) which is one-liquid type DNA ligation reagent.

(b) A cDNA encoding hT1R3 is inserted into a multicloning site (base number 895-1010) of the vector prepared in the step (a).
   This step (b) can be implemented, for example, according to the following substeps (b1) to (b8).
(b1) A sense primer and an antisense primer having a recognition sequence (5'-GGCGCGCC-3') of Asc I and a recognition sequence (5'-GCGGCCGC-3') of Not I immediately before and immediately after a coding region of hT1R3, respectively, are designed and prepared.
(b2) Using the sense primer and the antisense primer prepared in (b1), and employing a sequence comprising a cDNA sequence encoding hT1R3 as a template, PCR is performed to amplify a cDNA encoding hT1R3.
(b3) The cDNA fragment encoding hT1R3 obtained in (b2) is digested with restriction enzymes Asc I and Not I.
(b4) pEAK10 (Edge Biosystems) is digested with restriction enzymes Asc I and Not I.
(b5) The cDNA fragment encoding hT1R3 obtained in (b3) and pEAK10 (Edge Biosystems) obtained in (b4) are connected by a ligation reaction with Ligation high Ver.2 (TOYOBO) to insert a cDNA encoding hT1R3 into pEAK10 (Edge Biosystems).
(b6) pEAK10 (Edge Biosystems) obtained in (b5) is digested with restriction enzymes Hind III and Not I, and DNA fragments are separated by agarose electrophoresis, and a cDNA fragment of hT1R3 is purified.
(b7) The vector prepared in (a5) is digested with Hind III and Not I.
(b8) The cDNA fragment encoding hT1R3 obtained in (b6) and the vector obtained in (b7) are connected by a ligation reaction with Ligation high Ver.2 (TOYOBO), thereby, a cDNA encoding hT1R3 is inserted into a multicloning site of the vector prepared in (a5).

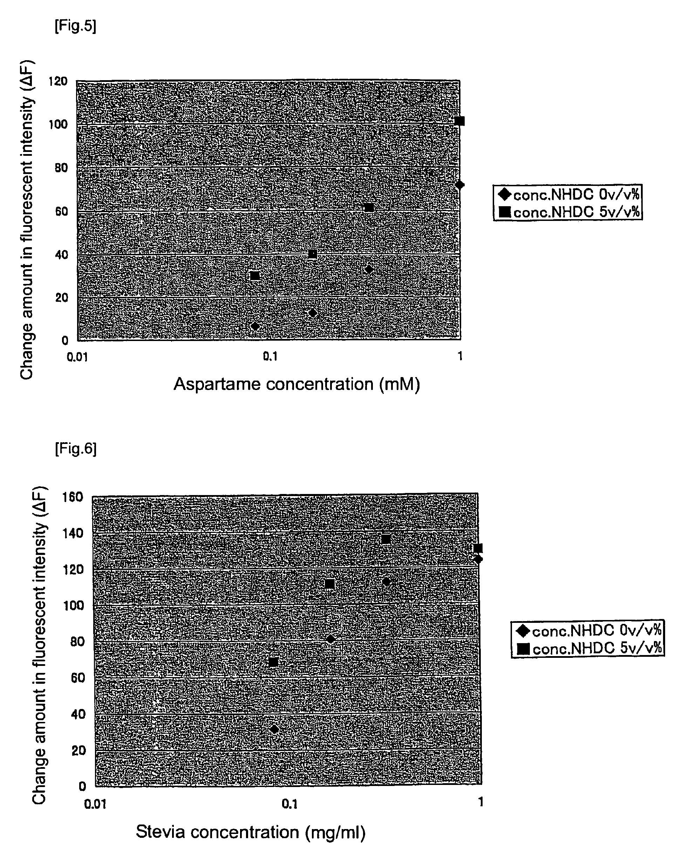

(c) Into pBluescript II SK (-) are inserted an IRES sequence, and a cDNA encoding hG16gust44, to prepare a vector (IRES2-hG16gust44/pBluescript II SK (-)) having a sequence in which these are connected (IRES2-hG16gust44 sequence) (see Fig. 5). The IRES sequence means a sequence encoding an internal ribosome entry site.
   This step (c) can be implemented, for example, according to the following substeps (c1) to (c11).
(c1) A sense primer having a recognition sequence (5'-CGGCCG-3') of Eco521 immediately before an IRES sequence is designed and prepared. On the other hand, an antisense primer corresponding to a part at which an IRES sequence terminates is designed and prepared.
(c2) Using the sense primer and the antisense primer prepared in (c1), and employing pIRES2-EGFP (Clontech) as a template, PCR is performed to amplify an IRES sequence. In addition, EGFP is an abbreviation of Enhanced Green Fluorescence Protein.
(c3) A sense primer and an antisense primer having a recognition sequence (5'-GCGGCCGC-3') of Not I immediately before and immediately after a coding region of hG16gust44 are designed and prepared, and PCR is performed employing a sequence comprising a cDNA sequence encoding hG16gust44 as a template, to amplify a cDNA encoding hG16gust44. The amplified fragment is digested with Not I.
(c4) pEAK10 (Edge Biosystems) is digested with Not I.
(c5) The cDNA fragment encoding hG16gust44 obtained in (c3), and pEAK10 (Edge Biosystems) obtained in (c4) are connected by a ligation reaction with Ligation high Ver.2 (TOYOBO), to prepare a vector (hG16gust44/pEAK10) in which a cDNA encoding hG16gust44 is inserted into pEAK10 (Edge Biosystems).
(c6) A sense primer of 18 bases comprising an initiation codon of hG16gust44 is designed and prepared. On the other hand, an antisense primer having a sequence in an hGH pA sequence is designed and prepared.
(c7) Employing hG16gust44/pEAK10 obtained in (c5) as a template, and using the sense primer and the antisense primer prepared in (c6), PCR is performed to amplify a cDNA encoding hG16gust44.
(c8) The IRES sequence obtained in (c2) is digested with Eco521.
(c9) The cDNA encoding hG16gust44 obtained in (c7) is digested with Not I.
(c10) pBluescript II SK (-) is digested with Not I.
(c11) The IRES sequence obtained in (c8), the cDNA fragment encoding hG16gust44 obtained in (c9), and pBluescript II SK (-) obtained in (c10) are connected by a ligation reaction with Ligation high Ver.2 (TOYOBO), to prepare IRES2-hG16gust44/pBluescript II SK (-).

(d) A sequence (IRES2-hG16gust44 sequence) in which an IRES sequence and a cDNA encoding hG16gust44 are connected, is inserted immediately after the DNA sequence encoding hT1R3 of the vector prepared in the step (b).
   This step (d) can be implemented, for example, according to the following substeps (d1) to (d3).
(d1) IRES2-hG16gust44/pBluescript II SK (-) prepared in (c11) is digested with Eco521, cDNA fragments are separated by agarose electrophoresis, and an IRES2-hG16gust44 sequence is purified.
(d2) A site present immediately after the DNA sequence encoding hT1R3 of the vector prepared in (b8) is digested with Not I.
(d3) The IRES2-hG16gust44 sequence obtained in (d1), and the vector obtained in (d2) are connected by a ligation reaction with Ligation high Ver.2 (TOYOBO), to insert the IRES2-hG16gust44 sequence immediately after the DNA sequence encoding hT1R3 of the vector prepared in (b8). As a result, pcDNA5/FRT comprising hT1R3-IRES2-hG16gust44 sequence is obtained.

(e) A cDNA encoding hT1R2 is inserted into pEAK10 (Edge Biosystems).
   This step can be implemented, for example, according to the following substeps (e1) to (e5).
(e1) A sense primer and an antisense primer having a recognition sequence (5'-GGCGCGCC-3') of Asc I and a recognition sequence (5'-GCGGCCGC-3') of Not I immediately before and immediately after a coding region of hT1R2, respectively, are designed and prepared.
(e2) Using the sense primer and the antisense primer prepared in (e1), and employing a sequence comprising a DNA sequence encoding hT1R2 as a template, PCR is performed to amplify a cDNA encoding hT1 R2.
(e3) The cDNA encoding hT1R2 obtained in (e2) is digested with Asc I and Not I.
(e4) pEAK10 (Edge Biosystems) is digested with Asc I and Not I.
(e5) The cDNA fragment encoding hT1R2 obtained in (e3), and the pEAK10 (Edge Biosystems) obtained in (e4) are connected by a ligation reaction with Ligation high Ver.2 (TOYOBO), to insert a cDNA encoding hT1R2 into pEAK10 (Edge Biosystems).

(f) An IRES2-hG16gust44 sequence is inserted immediately after the DNA sequence encoding hT1R2 of the vector prepared in the step (e).
   This step can be implemented, for example, according to the following substeps (f1) to (f3).
(f1) The IRES2-hG16gust44/pBluescript II SK (-) prepared in (c11) is digested with Eco521, cDNA fragments are separated by agarose electrophoresis, and an IRES2-hG16gust44 sequence is purified.
(f2) The vector obtained in (e5) is digested with Not I to cut a site present immediately after the cDNA encoding hT1R2 of.
(f3) The IRES2-hG16gust44 sequence obtained in (f1), and the vector obtained in (f2) are connected by a ligation reaction with Ligation high Ver.2 (TOYOBO), to insert an IRES2-hG16gust44 sequence immediately after the cDNA encoding hT1R2 of the vector obtained in (e5). As a result, pEAK10 (Edge Biosystems) comprising an hT1R2-IRES2-hG16gust44 sequence is obtained.

(g) The vector obtained in the step (d) is cut with EcoRV, and the hT1R2-IRES2-hG16gust44 sequence obtained in the steps (f) is inserted upstream of an hT1R3-IRES2-hG16gust44 sequence, to obtain an expression construct in which respective cDNAs encoding hT1R2 and hT1R3, and G protein α subunit are inserted into the same plasmid.
   A preferable example of the expression construct is an expression construct in which an EF-1α promoter-hT1R2-IRES2-hG16gust44-hGH pA sequence of the vector prepared in (f3) is inserted upstream of an hT1R3-IRES2-hG16gust44 sequence of the vector prepared in (d3), that is, an expression construct consisting of pcDNA5/FRT, having a sequence in which a cDNA encoding hT1R2 and a cDNA encoding hG16gust44 are connected so that an IRES sequence is flanked by the cDNAs, downstream of an EF-1α promoter, and in which a sequence in which a cDNA encoding hT1R3 and a cDNA encoding hG16gust44 so that an IRES sequence is flanked by the cDNAs, is inserted downstream of a CMV promoter present downstream of this sequence.
   This step (g) can be performed, for example, according to the following substeps (g1) to (g3).
(g1) A primer for conducting an In-Fusion reaction is designed and prepared, and using this and, employing the vector prepared in (f3) as a template, a region of EF-1α promoter-hT1R2-IRES2-hG16gust44-hGH pA is amplified by PCR. The primer is designed so that about 15 nucleotides homologous with an end of the vector obtained in (d3) which has been linearized with a restriction enzyme are added to a DNA fragment of the region.
(g2) The vector obtained in (d3) is digested with EcoRV.
(g3) The EF-1α promoter-hT1R2-IRES2-hG16gust44-hGH pA sequence fragment obtained in (g1), and the vector obtained in (g2) are connected using an In-Fusion Advantage PCR Cloning Kit (Clontech) to allow an expression construct in which an hT1R2-IRES2-hG16gust44 sequence of the vector prepared in (f3) is inserted upstream of an hT1R3-IRES2-hG16gust44 sequence of the vector obtained in (d3). For ligation with the In-Fusion Advantage PCR Cloning Kit (Clontech), the EF-1α promoter-hT1R2-IRES2-hG16gust44-hGH pA sequence fragment obtained in (g1), and the vector obtained in (G2) are mixed, an In-Fusion enzyme and a predetermined buffer are added, and a reaction is performed usually at 37°C for 15 minutes and, then, at 50°C for 15 minutes. According to the In-Fusion Advantage PCR Cloning Kit (Clontech), an objective DNA fragment can be cloned without undergoing limitation of a restriction enzyme site, and even in the case of a long chain DNA fragment.
By the above method, an expression construct necessary for obtaining a cultured cell strain used upon identification of the human sweet taste receptor-acting sweet taste regulating substance of the present invention is obtained.

Then, a method of preparing a cultured cell strain used for identifying the human sweet taste receptor acting sweet taste regulating substance of the present invention will be explained.
Preparation of the cultured cell strain is performed by cotransfecting the expression construct, and pOG44 being an Flp recombinase expression vector, into a 293 cell in which an FRT site has been incorporated into one place in a genome DNA, using an Flp-In system (Invitrogen). According to the Flp-In system (Invitrogen), an FRT site harbored in a genome DNA of a 293 cell is cleaved with a transiently expressed Flp recombinase, a foreign gene is introduced into that portion, and the gene is expressed. That is, respective genes encoding hT1R2 and hT1R3, and hG16gust44 are inserted into an FRT site of a chromosome of a 293 cell by site-specific recombination utilizing an Flp recombinase derived from Saccharomyes cerevisiae, and an FRT site being a target site of the Flp recombinase. As a result, a cultured cell strain stably expressing hT1R2 and hT1R3, and hG16gust44 is obtained rapidly and effectively. In this cultured cell strain, respective genes encoding hT1R2 and hT1R3, and hG16gust44 are transcribed into two mRNAs and, thereafter, translated into three proteins, that is, hT1R2 and hT1R3, and hG16gust44.

A 293 cell in which an FRT site is incorporated into one place in a genome DNA can be prepared, for example, as follows.
First, a pFRT/lacZeo being a vector for introducing an FRT site is gene-transferred into a 293 cell being a host cell, and an introduced cell is selected with Zeocin. Since pFRT/lacZeo has a fused gene of lacZ and a Zeocin-resistant gene, a cell in which pFRT/lacZeo has been introduced into a genome expresses β-galactosidase, and becomes Zeocin-resistant. An extent of Zeocin sensitivity can be confirmed by a β-galactosidase assay.
Then, a Southern blot method for a lacZ gene is performed. Thereby, it is confirmed that an FRT site is incorporated into only one place of a position being capable of transcribed in a genome. It is convenient to use a commercially available Flp-In-293 cell (Invitrogen), as a 293 cell in which an FRT site has been incorporated into one place in a genome DNA.

Preparation of a cultured cell strain used for identifying the human sweet taste receptor-acting sweet taste regulating substance of the present invention can be performed by cotransfecting the expression construct and pOG44 being an expression vector of an Flp recombinase into a 293 cell in which an FRT site has been incorporated into one place, and selecting a hygromycin B-resistant cell strain in a selection medium containing hygromycin B.

The Flp recombinase is one kind of enzymes which conduct a site-specific recombination reaction, and pOG44 which is an expression vector of the enzyme is known. When the expression construct and pOG44 are cotransfected, since the expression construct is inserted into an FRT site of the 293 cell with an expressed Flp recombinase, and the 293 cell is changed into hygromycin B-resistant and Zeocin-sensitive, whether an objective gene has been inserted or not can be easily determined, by using these drug resistances as an index.

It is possible to appropriately select, as a method of cotransfection, known methods, for example, a lipofection method, an electroporation method, a calcium phosphate-DNA precipitation method, a calcium chloride method, a calcium chloride/rubidium chloride method, a liposome method, a DEAE-dextran method, a microinjection method and the like.

As described above, the expression construct is placed into the same position in a genome, and a cultured cell strain expressing hT1R2 and hT1R3, and hG16gust44 simultaneously can be obtained. Expression of hT1R2 and hT1R3, and hG16gust44 can be confirmed by extracting proteins from the cultured cell strain, and performing a Western blot method using an hT1R2 antibody, an hT1R3 antibody, and an antibody capable of recognizing a chimeric protein hG16gust44.

The stable expression cell is proliferated and/or maintained by culturing it in a nutrient medium. A specific method of proliferating and/or maintaining the stable expression cell may be appropriately determined, but in order to minimize desensitization with glucose, it is preferable to perform proliferation and/or maintenance at 37°C, for example, using a low glucose (1.000 mg/ml) Dulbecco's modified Eagle (DMEM) medium supplemented with L-glutamine to 4 mM (Virology, Vol.8, p. 396, 1959), to which 10% HI-FBS (Heat Inactivated Fetal Bovine Serum) and 100 µg/ml hygromycin B (Invitrogen) have been added.

The human sweet taste receptor-acting sweet taste regulating substance of the present invention is identified by utilizing the stable cultured cell strain, contacting a certain specified sweet taste substance with the cultured cell strain, measuring a physiological response generated by the contact, then, contacting the cultured cell strain with the sweet taste substance with a candidate substance of a human sweet taste receptor-acting sweet taste regulating substance added thereto, measuring a physiological response generated by the contact, and comparing both measured results.

For screening a candidate substance of a human sweet taste receptor-acting sweet taste regulating substance for identifying the human sweet taste receptor-acting sweet taste regulating substance of the present invention; for example, the screening is performed as follows.
First, a cultured cell strain expressing the expression construct is obtained as described above, then, the predetermined number of the cultured cells are seeded on each well (e.g. 10,000 to 500,000 cells/well) of a microplate having many wells (24 wells, 48 wells, 96 wells, 384 wells, etc.), and are cultured for 1 day in a predetermined medium (e.g. DMEM medium).
Thereafter, in each case where a specified sweet taste substance, or a sweet taste substance and a candidate substance of a human sweet taste receptor-reacting sweet taste regulating substance are added, a physiological response generated in the stable cultured cell strain is measured, a measured value when a sweet taste substance is alone, and a measured value when a candidate substance is added are compared, and a human sweet taste receptor-reacting sweet taste regulating substance to a sweet taste substance is identified, based on an extent of that difference. The candidate substance includes an artificially prepared arbitrary substance, in addition to a substance present in the natural world.

When a physiological response generated in the cultured cell strain is measured, phenomenon which is changed with activation of a sweet taste receptor is appropriately selected as the physiological response. When a sweet taste receptor is activated, thereafter, a variety of phenomena are initiated in cells. When a taste substance is bound to a receptor, a calcium concentration in cells is raised via an information transduction process through a second messenger (IP₃ (inositol triphosphate), DAG) and the like in cells. Therefore, examples of the physiological response being a measurement subject include change in a second messenger in cells and change in a calcium concentration in cells, which are changed with activation of a sweet taste receptor.

In the present invention, it is preferable that measurement of the physiological response to a sweet taste substance is performed by a calcium imaging method of observing change in a calcium concentration in cells induced by sweet taste stimulation from outside of cells using a fluorescent calcium indicator. Thereby, objective assessment of the sweet taste enhancing effect or suppressing effect at a receptor level becomes possible, unlike the previous sensory evaluation. By digitalizing an extent of sweet taste enhancement or suppression, the sweet taste enhancing effect or suppressing effect of various substances can be compared mutually.
The measured result when a sweet taste substance is alone, and the measurement result when a candidate substance of a human sweet taste receptor-acting sweet taste regulating substance is added are compared, and a human sweet taste receptor-acting sweet taste regulating substance to a sweet taste substance is identified based on an extent of that difference. Regarding an extent of a difference at which a substance is determined to be a human sweet taste receptor-acting sweet taste regulating substance of the present invention, for example, in the case by a calcium imaging method, it can be determined to be a human sweet taste receptor-acting sweet taste regulating substance of the present invention when a statistically significant difference is recognized in the measured cell response.

Since the fluorescent calcium indicator is required that fluorescent property is changed in a calcium concentration which can be physiologically changed, and change at that time is induced calcium-specifically, currently, a compound having a structure in which a complex forming site and a fluorescent chromophore are bound, is generally used as the fluorescent indicator. In the present invention, it is preferable to use Fluo-4 AM, and Fura-2 AM which are such a fluorescent calcium indicator.

In the present invention, it is preferable to verify whether sweet taste regulating action actually occurs at a level of a human sweet taste receptor or not, by digitalizing and visualizing a physiological response to a sweet taste substance, using a calcium imaging method. For doing so, for example, a cell response is digitalized by a simultaneous assay with a multiplate reader and, furthermore, a change in a calcium concentration in cells is imaged by imaging using a microscope, and it is observed whether or not each cell is responding. By performing microscope observation using a fluorescent indicator different from the fluorescent indicator used in a simultaneous assay with a multiplate reader, it can be confirmed that a cell response is not due to an artifact.

The simultaneous assay with a multiplate reader may be appropriately performed according to the known method. However, it is simple and rapid to perform automated fluorescent calcium imaging using FlexStation 3 (Molecular Devices), and a high throughput assay becomes possible. FlexStation 3 is a multiplate reader in which performance of SpectraMax M5e (Molecular Devices), and an 8 channel pipetter are fused.

Automated fluorescent imaging using FlexStation 3 (Molecular Devices) can be performed, for example, according to the following procedure.
First, cells are suspended in a low glucose (1,000 mg/ml) DMEM medium from which hygromycin B has been removed, and each 70 to 80 thousands of cells are seeded on each well of a 96-well plate (Corning, CellBIND Surface).

Then, after cultured at 37°C for 24 hours, a medium is removed, and replaced with a suitable amount of a HEPES (2-[4-(2-hydroxyethyl)-1-piperazinyl]ethanesulfonic acid) buffer, and a HEPES buffer containing a fluorescent calcium indicator (Fluo-4 AM attached to Molecular Devices, FLIPR Ca 4 Assay Kit) is further added. In order to facilitate transfer into cells, a fat-soluble acetoxymethyl group has been introduced into the fluorescent indicator Fluo-4 (excitation wavelength: 495 nm, fluorescent wavelength: 518 nm), and when it is added to a medium, it is easily taken into cells, and is hydrolyzed with an esterase in cells. Hydrolyzed Fluo-4 becomes difficult to be permeated through a cell membrane, and is diffused into cells to form a complex with calcium, emitting intense fluorescence. In a 96-well plate for fluorescent observation, there are a plastic bottom and a film bottom. Since a plate of the plastic bottom is satisfactory in adherability and growth of cells, it is used at a simultaneous assay using a multiplate reader, for observing an entire well. However, since a plate of this plastic bottom is bad in transmission property of a UV wavelength, and inhibits transmission of excited light of Fura-2, Fluo-4 is used.

Then, after incubated at 27 to 37°C for 30 to 60 minutes, by adding thereto a sweet taste substance at a particular concentration or a solution of a sweet taste substance and a substance to be tested, taste stimulation is performed at 27 to 37°C.
By measuring a fluorescent reaction (excitation at 485 nm, fluorescence at 525 nm) from immediately after addition of a sweet taste substance or a sweet taste substance and a substance to be tested to after 60 to 120 seconds, a response of a sweet taste receptor-expressing cell to sweet taste stimulation can be quantitated.

Fluorescent calcium imaging using a fluorescent microscope can be performed according to the following procedure.
First, cells are suspended in a low glucose (1,000 mg/ml) DMEM medium, from which hygromycin B has been removed, and each 40 to 80 thousands cells are seeded on each well of a 96-well plate (Greiner, Lumox).

Then, after cultured at 37°C for 24 to 48 hours, a medium is removed, and is replaced with a suitable amount of a HEPES buffer, and a HEPES buffer containing a fluorescent calcium indicator (Fura-2 AM) is further added.

After incubated at 27 to 37°C for 30 to 60 minutes, an extracellular fluorescent indicator is removed, and this is finally replaced with a suitable amount of a HEPES buffer, and is allowed to stand at room temperature for 10 to 20 minutes. By adding thereto a sweet taste substance at a particular concentration or a solution of a sweet taste substance and a substance to be tested, taste stimulation is performed at room temperature. In the fluorescent indicator Fura-2 (excitation wavelength: 340 nm/380 nm, fluorescent wavelength: 510 nm), a fluorescent intensity of excitation at 340 nm is increased, and a fluorescent intensity of excitation at 380 nm is reduced when a calcium ion concentration is increased.

Then, a fluorescent image (excitation at 340 nm, 380 nm, fluorescence at 510 nm) in the field of a microscope is taken in from immediately after addition of a sweet taste substance or a solution of a sweet taste substance and a substance to be tested to after 60 to 300 seconds, and a ratio of 2-wavelength excitation fluorescence is displayed with a pseudo color, thereby, a response of a sweet taste receptor-expressing cell to sweet taste stimulation can be observed.
As described above, in the present invention, universal phenomenon not depending on an observation system can be observed upon measurement of a physiological response, by jointly using two kinds of the fluorescent indicators having different fluorescent properties in a calcium imaging method.

### Examples

The present invention will be explained in more detail below by way of Examples.

### (Example 1)

### Preparation of cultured cell strain which is allowed to functionally stably express both of a human sweet taste receptor (hT1R2+hT1R3) and a chimeric G protein (hG16gust44)

First, as an expression construct, pcDNA5/FRT (Invitrogen) (Fig. 2) having a sequence in which cDNA encoding hT1R2 and a cDNA encoding hG16gust44 are connected so that an IRES sequence is flanked by the cDNAs, downstream of an EF-1α promoter, and having a sequence in which a cDNA encoding hT1R3 and a cDNA encoding hG16gust44 are connected so that an IRES sequence is flanked by the cDNAs, downstream of a CMV promoter present downstream of this sequence, was prepared according to the following procedure.

A sense primer (SEQ ID NO:1: TATAGATCTGATATCCCCCTATGGTGCACTCTC) having a recognition sequence (5'-AGATCT-3') of Bgl II at a 5' end, and a recognition sequence of EcoRV immediately under therefrom, and an antisense primer (SEQ ID NO:2: TAGAAGGCACAGTCGAGG) having a sequence in a BGH pA sequence were designed and prepared.
Using these sense primer and antisense primer, and employing pcDNA5/FRT (Invitrogen) as a template, a polymerase chain reaction (PCR) was performed to amplify a DNA fragment comprising a sequence in which respective recognition sequences of Bgl II and EcoRV are connected. Then, this amplified DNA fragment was digested with Bgl II and Not I, and pcDNA5/FRT (Invitrogen) was digested with Bgl II and Not I. These restriction enzyme digestion products were connected by a ligation reaction using Ligation high Ver.2 (TOYOBO), thereby, a vector having a recognition sequence of EcoRV immediately under a recognition sequence of Bgl II of pcDNA5/FRT (Invitrogen) was prepared.

Then, a sense primer (SEQ ID NO:3: GATCGGCGCGCCGCCATGCTGGGCCCTGCTGTC) and an antisense primer (SEQ ID NO:4: TAGAAGGCACAGTCGAGG) having a recognition sequence (5'-GGCGCGCC-3') of Asc I and a recognition sequence (5'-GCGGCCGC-3') of Not I immediately before and immediately after a coding region of hT1R3, respectively, were designed and prepared.
Using these sense primer and antisense primer, and employing a sequence comprising a cDNA sequence encoding hT1R3 as a template, PCR was performed to amplify a cDNA encoding hT1R3.
The amplified cDNA fragment encoding T1R3 was digested with Asc I and Not I, and pEAK10 (Edge Biosystems) was digested with Asc I and Not I. These restriction enzyme digestion products were connected by a ligation reaction using Ligation high Ver.2 (TOYOBO) to insert a cDNA encoding hT1R3 into pEAK10 (Edge Biosystems). Then, this was digested with Hind III and Not I, DNA fragments were separated by agarose electrophoresis, and a cDNA fragment of hT1R3 was purified.
This cDNA fragment, and a vector having a recognition sequence of EcoRV immediately under a recognition sequence of Bgl II of pcDNA5/FRT (Invitrogen), which had been digested with Hind III and Not I, were connected by a ligation reaction using Ligation high Ver.2 (TOYOBO), thereby, a cDNA encoding hT1R3 was inserted into a multicloning site of the vector.

Then, a sense primer (SEQ ID NO:5: GATCCGGCCGGCCCCTCTCCCTCCCCCC) having a recognition sequence (5'-CGGCCG-3') of Eco521 immediately before an IRES sequence and an antisense primer (SEQ ID NO:6: GGTTGTGGCCATATTATC) corresponding to a part at which an IRES sequence terminates were designed and prepared.
Using these sense primer and antisense primer, and employing pIRES2-EGFP (Clontech) as a template, PCR was performed to amplify an IRES sequence.
A sense primer (SEQ ID NO: 7: GATCGCGGCCGCATGGCCCGCTCGCTGACC) and an antisense primer (SEQ ID NO: 8: GATCGCGGCCGCGAATTCACTAGTGATTTA) having a recognition sequence (5'-GCGGCCGC-3') of Not I immediately before and immediately after a coding region of hG16gust44 were designed and prepared. Employing a sequence comprising a cDNA sequence encoding hG16gust44 as a template, PCR was performed to amplify a cDNA encoding hG16gust44. The amplified fragment was digested with Not I, pEAK10 (Edge Biosystems) was digested with Not I, and these were connected by a ligation reaction using Ligation high Ver.2 (TOYOBO) to prepare a vector (hG16gust44/pEAK10) in which a cDNA encoding hG16gust44 is inserted into pEAK10 (Edge Biosystems).
Then, a sense primer (SEQ ID NO:9: ATGGCCCGCTCGCTGACC) of 18 bases comprising an initiation codon of hG16gust44, and an antisense primer (SEQ ID NO:10: CTGGATGCAGGCTACTCTA) having a sequence in an hGH pA sequence were designed and prepared.
Using these sense primer and antisense primer, and employing hG16gust44/pEAK10 as a template, PCR was performed to amplify a cDNA encoding hG16gust44.
Then, the IRES sequence digested with Eco521, the cDNA encoding hG16gust44 digested with Not I, and pBluescript II SK (-) digested with Not I were connected by a ligation reaction using Ligation high Ver.2 (TOYOBO) to prepare IRES2-hG16gust44/pBluescript II SK (-).

Then, the above-mentioned IRES2-hG16gust44/pBluescript II SK (-) was digested with Eco521, DNA fragments were separated by agarose electrophoresis, and an IRES2-hG16gust44 sequence was purified.
The pcDNA5/FRT (Invitrogen) in which a cDNA encoding hT1R3 had been inserted into a multicloning site was digested with Not I to cut a part present immediately after a DNA sequence encoding hT1R3 in the vector. This vector and the above-mentioned IRES2-hG16gust44 sequence were connected by a ligation reaction using Ligation high Ver.2 (TOYOBO) to obtain pcDNA5/FRT comprising an hT1R3-IRES2-hG16gust44 sequence, in which an IRES2-hG16gust44 sequence is inserted immediately after a DNA sequence encoding hT1R3.

Then, a sense primer (SEQ ID NO:11: GATCGGCGCGCCGCCATGGGGCCCAGGGCAAAG) and an antisense primer (SEQ ID NO:12: GATCGCGGCCGCCTAGTCCCTCCTCATGGT) having a recognition sequence (5'-GGCGCGCC-3') of Asc I and a recognition sequence (5'-GCGGCCGC-3') of Not I immediately before and immediately after a coding region of hT1R2, respectively, were designed and prepared.
Using these sense primer and antisense primer, and employing a sequence comprising a DNA sequence encoding hT1R2 as a template, PCR was performed to amplify a cDNA encoding hT1R2. The resulting cDNA encoding hT1R2 was digested with Asc I and Not I, pEAK10 (Edge Biosystems) was digested with Asc I and Not I, and these were connected by a ligation reaction using Ligation high Ver.2 (TOYOBO) to insert a cDNA encoding hT1R2 into pEAK10 (Edge Biosystems).

Then, the above-mentioned IRES2-hG16gust44/pBluescript II SK (-) was digested with Eco521, cDNA fragments were separated by agarose electrophoresis, and an IRES2-hG16gust44 sequence was purified.
pEAK10 (Edge Biosystems) in which a cDNA encoding hT1R1 had been inserted, was digested with Not I to cut a part present immediately after a cDNA encoding hT1R2 in the vector. This vector and the above-mentioned IRES2-hG16gust44 sequence were connected by a ligation reaction using Ligation high Ver.2 (TOYOBO) to insert an IRES2-hG16gust 44 sequence immediately after a cDNA encoding hT1R2, to obtain pEAK10 (Edge Biosystems) comprising an hT1R2-IRES2-hG16gust44 sequence.

Then, primers for performing an In-Fusion reaction (SEQ ID NO:13: ATCGGGAGATCTGATGCATAACTAGTGAGGCTC and SEQ ID NO:14: GCACCATAGGGGGATAGCGGATCCAGACATGAT) were designed and prepared. Using them, and employing the above-mentioned pEAK10 (Edge Biosystems) comprising an hT1R2-IRES2-hG16gust44 sequence as a template, a region of EF-1 α promoter-hT1R2-IRES2-hG16gust44-hGH pA was amplified by PCR.
This EF-1 α promoter-hT1R2-IRES2-hG16gust44-hGH pA sequence fragment, and pcDNA5/FRT comprising an hT1R3-IRES2-hG16gust44 sequence digested with EcoRV were connected by using In-Fusion Advantage PCR Cloning Kit (Clontech) to prepare an expression construct in which an hT1R2-IRES2-hG16gust44 sequence is inserted upstream of an hT1R3-IRES2-hG16gust44 sequence.

Then, 0.8 µg of the expression construct and 7.2 µg of pOG44 were transfected into two million Flp-In-293 cells (Invitrogen) by a lipofection method. From two days after transfection, a hygromycin-resistant cell was selected by a medium containing 100 µg/ml hygromycin B (Invitrogen). By continuing culturing using the similar medium, a cultured cell strain functionally stably expressing both of a human sweet taste receptor (hT1 R2+hT1 R3) and a G protein α subunit, used for identifying a human sweet taste receptor-acting sweet taste regulating substance in the following Examples, was established.
Thereafter, this cultured cell strain was proliferated and maintained at 37°C in a low glucose (1.000 mg/ml) Dulbecco's modified Eagle (DMEM) medium containing 10% HI-FBS (Heat Inactivated Fetal Bovine Serum), 100 µg/ml hygromycin B (Invitrogen), and 4 mM L-glutamine.

### (Example 2)

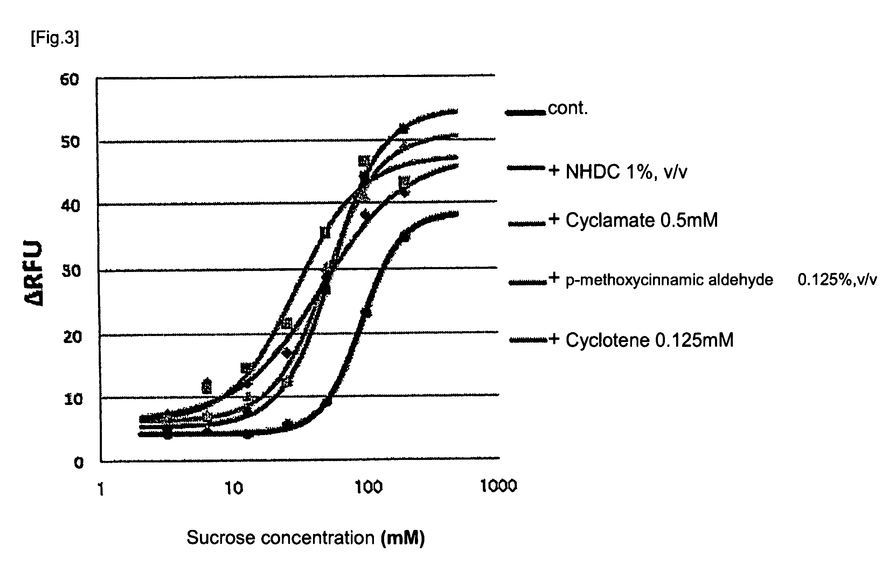

Physiological response of sweet taste receptor-expressing cell to sucrose stimulation, when neohesperidin dihydrochalcone (NHDC) is added
How a physiological response of a sweet taste receptor-expressing cell by sucrose changes by adding neohesperidin dihydrochalcone was analyzed by automated fluorescent imaging.
The sweet taste receptor-expressing cell obtained in Example 1 was trypsinized, and suspended in the DMEM medium, a cell density was measured, and each about 80 thousands of cells were seeded on each well of a 96-well plate (Corning, Cell BIND Surface).
After cultured at 37°C for 24 hours, the medium was removed, and replaced with 50 µl of a HEPES buffer, and 50 µg of a HEPES buffer containing a fluorescent calcium indicator (Fluo-4 AM attached to Molecular Devices, FLIPR Ca 4 Assay Kit) was further added. Then, this was incubated at 27°C for 45 minutes to prepare a cell to be subjected to automated fluorescent imaging.
Then, to the cells was added a HEPES buffer containing sucrose, so that a final concentration of sucrose became a concentration described in Table 1, and sucrose stimulation was performed at 27°C.
A fluorescent reaction (excitation at 485 nm, fluorescence at 525 nm) from immediately after addition of a HEPES buffer containing sucrose to after 100 seconds was measured using FlexStation 3 (Molecular Devices), and a response of a sweet taste receptor-expressing cell to sucrose stimulation was quantitated by automated fluorescent imaging. Results are shown in Table 1 and Fig. 3.

In the same manner as that described above, a HEPES buffer containing sucrose was added to a cell to be subjected to automated fluorescent imaging, so that a final concentration of sucrose became a concentration described in Table 2, a HEPES buffer containing conc. neohesperidin dihydrochalcone was further added so that a final concentration of neohesperidin dihydrochalcone became 2 v/v%, stimulation was performed at 27°C, a fluorescent reaction (excitation at 485 nm, fluorescence at 525 nm) from immediately after addition of the solution to after 100 seconds was measured by using FlexStation 3 (Molecular Devices), and a response of a sweet taste receptor-expressing cell when neohesperidin dihydrochalcone was added to sucrose was quantitated. Results are shown in Table 2 and Fig. 3. The vertical axis of Fig. 3 is a maximum (ΔF) of a change amount in a fluorescent intensity between immediately after stimulation and after 100 seconds from stimulation, that is, a response intensity of a cell, and the horizontal axis indicates a sucrose concentration (mM) as expressed by logarithm.

From the obtained results, it was recognized that a physiological response by sucrose is synergistically raised by adding neohesperidin dihydrochalcone, in a cultured cell strain which is allowed to functionally stably express both of a human sweet taste receptor (hT1 R2+hT1 R3) and a chimeric G protein (hG16gust44).

**[Table 1]**

| Sucrose (mM) | Change amount in fluorescent intensity (ΔF) |
|---|---|
| 0 | 3.9 |
| 3.125 | 4.2 |
| 6.25 | 4.6 |
| 12.5 | 4.1 |
| 25 | 5.7 |
| 50 | 9.2 |
| 100 | 23.3 |
| 200 | 34.9 |

**[Table 2]**

| Sucrose (mM) | NHDC (V/V%) | Change amount in fluorescent intensity (ΔF) |
|---|---|---|
| 0 | 1 | 4.6 |
| 3.125 | 1 | 5.1 |
| 6.25 | 1 | 6.9 |
| 12.5 | 1 | 7.6 |
| 25 | 1 | 12.4 |
| 50 | 1 | 26.7 |
| 100 | 1 | 44.3 |
| 200 | 1 | 51.8 |

### (Example 3)

### Physiological response of sweet taste receptor-expressing cell to sucrose stimulation, when cyclamate is added

How a physiological response of a sweet taste receptor-expressing cell by sucrose changes by adding cyclamate was analyzed by automated fluorescent imaging.
In the same manner as in Example 2, a HEPES buffer containing sucrose was added to a cell to be subjected to automated fluorescent imaging, so that a final concentration of sucrose became a concentration described in Table 3, a HEPES buffer containing cyclamate was further added so that a final concentration of cyclamate became 1 mM, stimulation was performed at 27°C, a fluorescent reaction (excitation at 485 nm, fluorescence at 525 nm) from immediately after addition of the solution to after 100 seconds was measured using FlexStation 3 (Molecular Devices), and a response of a sweet taste receptor-expressing cell when cyclamate was added to sucrose was quantitated. Results are shown in Table 3 and Fig. 3.

From the obtained results, it was recognized that a physiological response by sucrose is synergistically raised by adding cyclamate, in a cultured cell strain which is allowed to functionally stably express both of a human sweet taste receptor (hT1 R2+hT1 R3) and a chimeric G protein (hG16gust44).

**[Table 3]**

| Sucrose (mM) | Cyclamate (mM) | Change amount in fluorescent intensity (ΔF) |
|---|---|---|
| 0 | 0.5 | 4.5 |
| 3.125 | 0.5 | 7.4 |
| 6.25 | 0.5 | 7.2 |
| 12.5 | 0.5 | 10.2 |
| 25 | 0.5 | 12.6 |
| 50 | 0.5 | 30.3 |
| 100 | 0.5 | 40.9 |
| 200 | 0.5 | 49.1 |

### (Example 4)

### Physiological response of sweet taste receptor-expressing cell to sucrose stimulation, when p-methoxycinnamic aldehyde is added

How a physiological response of a sweet taste receptor-expressing cell by sucrose changes by adding p-methoxycinnamic aldehyde was analyzed by automated fluorescent imaging.
In the same manner as in Example 2, a HEPES buffer containing sucrose was added to a cell to be subjected to automated fluorescent imaging, so that a final concentration of sucrose became a concentration described in Table 4, a HEPES buffer containing conc. p-methoxycinnamic aldehyde was further added so that a final concentration of p-methoxycinnamic aldehyde became 0.25 v/v%, stimulation was performed at 27°C, a fluorescent reaction (excitation at 485 nm, fluorescence at 525 nm) from immediately after addition of the solution to after 100 seconds was measured by using FlexStation 3 (Molecular Devices), and a response of a sweet taste receptor-expressing cell when p-methoxycinnamic aldehyde was added to sucrose was quantitated. Results are shown in Table 4 and Fig. 3.

From the obtained results, it was recognized that a physiological response by sucrose is synergistically raised by adding p-methoxycinnamic aldehyde, in a cultured cell strain which is allowed to functionally stably express both of a human sweet taste receptor (hT1 R2+hT1 R3) and a chimeric G protein (hG16gust44).

**[Table 4]**

| Sucrose (mM) | p-methoxycinnamic aldehyde (v/v%) | Change amount in fluorescent intensity (ΔF) |
|---|---|---|
| 0 | 0.125 | 4.6 |
| 3.125 | 0.125 | 7.5 |
| 6.25 | 0.125 | 12.4 |
| 12.5 | 0.125 | 12.2 |
| 25 | 0.125 | 17.0 |
| 50 | 0.125 | 28.7 |
| 100 | 0.125 | 38.1 |
| 200 | 0.125 | 41.4 |

### (Example 5)

### Physiological response of sweet taste receptor-expressing cell to sucrose stimulation, when cyclotene is added

How a physiological response of a sweet taste receptor-expressing cell by sucrose changes by adding cyclotene was analyzed by automated fluorescent imaging.
In the same manner as in Example 2, a HEPES buffer containing sucrose was added to a cell to be subjected to automated fluorescent imaging, so that a final concentration of sucrose became a concentration described in Table 5, a HEPES buffer containing cyclotene was further added so that a final concentration of cyclotene became 0.25 mM, stimulation was performed at 27°C, a fluorescent reaction (excitation at 485 nm, fluorescence at 525 nm) from immediately after addition of the solution to after 100 seconds was measured by using FlexStation 3 (Molecular Devices), and a response of a sweet taste receptor-expressing cell when cyclotene was added to sucrose was quantitated. Results are shown in Table 5 and Fig. 3.

From the obtained results, it was recognized that a physiological response by sucrose is synergistically raised by adding cyclotene, in a cultured cell strain which is allowed to functionally stably express both of a human sweet taste receptor (hT1 R2+hT1 R3) and a chimeric G protein (hG16gust44).

**[Table 5]**

| Sucrose (mM) | Cyclotene (mM) | Change amount in fluorescent intensity (ΔF) |
|---|---|---|
| 0 | 0.125 | 4.4 |
| 3.125 | 0.125 | 6.6 |
| 6.25 | 0.125 | 11.5 |
| 12.5 | 0.125 | 14.7 |
| 25 | 0.125 | 21.6 |
| 50 | 0.125 | 35.6 |
| 100 | 0.125 | 46.6 |
| 200 | 0.125 | 43.2 |

Any of concentrations of respective substances tested in Examples 2 to 5 is not higher than concentrations at which a cell response is observed when the substances are administered alone in all cases, but a sensitivity of a cell response to sucrose could be synergistically raised.
In addition, since sucrose is weak in an extent of a sweet taste, unless added at a concentration of around 50 mM, a response of a sweet taste receptor cell is not observed. However, conversely, when a sucrose solution at a concentration exceeding 200 mM is added, since cells undergo influence of an osmotic pressure, it becomes difficult to measure a normal cell response. Therefore, usually, possible digitalization of a sweet degree of sucrose in a sweet taste assay system using a cell is limited to a narrow range of about 50 to 200 mM, but it becomes possible to further lower a lower limit of this limit value when the cell line and the human sweet taste receptor-acting sweet taste enhancing substances are utilized.

### (Example 6)

### Physiological response of sweet taste receptor-expressing cell to sucrose stimulation, when neohesperidin dihydrochalcone is added

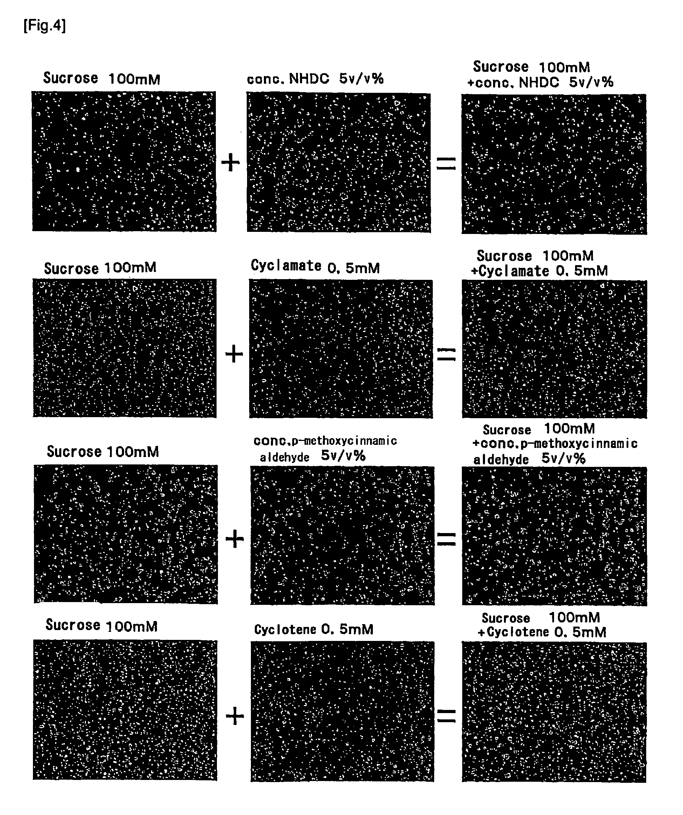

How a physiological response of a sweet taste receptor-expressing cell by sucrose changes by adding neohesperidin dihydrochalcone was analyzed by fluorescent calcium imaging using a fluorescent microscope.
First, the sweet taste receptor-expressing cell obtained in Example 1 was suspended in a low glucose (1.000 mg/ml) DMEM medium, from which hygromycin B had been removed, and each about 80 thousands of cells were seeded on each well of a 96-well plate (Greiner, Lumox).
After cultured at 37°C for 24 hours, the medium was removed, and replaced with 50 µl of a HEPES buffer, and 50 µl of a HEPES buffer containing a fluorescent calcium indicator (Fura-2 AM) was further added.
After incubated at 27°C for 30 minutes, an extracellular fluorescent indicator was removed, and was finally replaced with 100 µl of a HEPES buffer, and this was allowed to stand at room temperature for 15 minutes to prepare a cell to be subjected to fluorescent calcium imaging using a fluorescent microscope.
A HEPES buffer containing sucrose was added to this cell, so that a final concentration of sucrose became 100 mM, thereby, sucrose stimulation was performed at room temperature.
A fluorescent image (excitation at 340 nm and 380 nm, fluorescence at 510 nm) in the field of a microscope after 30 seconds from addition of the sucrose solution was taken, and a cell response by sucrose was observed by displaying a ratio of 2-wavelength excitation fluorescence by a pseudocolor. Results are shown in Fig. 4.

In the same manner as that described above, stimulation was performed at room temperature by adding a HEPES buffer containing conc. neohesperidin dihydrochalcone to a cell to be subjected to fluorescent calcium imaging, so that a final concentration of neohesperidin dihydrochalcone became 5 v/v%, and the state of the cell was observed by a fluorescent image. Results are shown in Fig. 4.

In the same manner as that described above, stimulation was performed at room temperature by adding a HEPES buffer containing sucrose to a cell to be subjected to fluorescent calcium imaging, so that a final concentration of sucrose became 100 mM, and further adding a HEPES buffer containing conc. neohesperidin dihydrochalcone, so that a final concentration of neohesperidin dihydrochalcone became 5 v/v%, and the state of the cell was observed by a fluorescent image. Results are shown in Fig. 4.

From the obtained results, it could be visually confirmed that a physiological response by sucrose is increased by adding neohesperidin dihydrochalcone, in a cultured cell strain which is allowed to functionally stably express both of a human sweet taste receptor (hT1 R2+hT1 R3) and a chimeric G protein (hG16gust44).

### (Example 7)

### Physiological response of sweet taste receptor-expressing cell to sucrose stimulation, when cyclamate is added

How a physiological response of a sweet taste receptor-expressing cell by sucrose changes by adding cyclamate was analyzed by fluorescent calcium imaging using a fluorescent microscope.

In the same manner as in Example 6, stimulation was performed at room temperature by adding a HEPES buffer containing sucrose to a cell to be subjected to fluorescent calcium imaging, so that a final concentration of sucrose became 100 mM. A fluorescent image (excitation at 340 nm and 380 nm, fluorescence at 510 nm) in the field of a microscope after 30 seconds from addition of the sucrose solution was taken, and a cell response by sucrose was observed by displaying a ratio of 2-wavelength excitation fluorescence by a pseudocolor. Results are shown in Fig. 4.

In the same manner as in Example 6, stimulation was performed at room temperature by adding a HEPES buffer containing cyclamate to a cell to be subjected to fluorescent calcium imaging, so that a final concentration of cyclamate became 0.5 mM, and the state of the cell was observed by a fluorescent image. Results are shown in Fig. 4.

In the same manner as in Example 6, stimulation was performed at room temperature by adding a HEPES buffer containing sucrose to a cell to be subjected to fluorescent calcium imaging, so that a final concentration of sucrose became 100 mM, and further adding a HEPES buffer containing cyclamate, so that a final concentration of cyclamate became 0.5 mM, and the state of the cell was observed by a fluorescent image. Results are shown in Fig. 4.

From the obtained results, it could be visually confirmed that a physiological response by sucrose is increased by adding cyclamate, in a cultured cell strain which is allowed to functionally stably express both of a human sweet taste receptor (hT1R2+hT1R3) and a chimeric G protein (hG16gust44).

### (Example 8)

### Physiological response of sweet taste receptor-expressing cell to sucrose stimulation, when p-methoxycinnamic aldehyde is added

How a physiological response of a sweet taste receptor-expressing cell by sucrose changes by adding p-methoxycinnamic aldehyde was analyzed by fluorescent calcium imaging using a fluorescent microscope.

In the same manner as in Example 6, stimulation was performed at room temperature by adding a HEPES buffer containing sucrose to a cell to be subjected to fluorescent calcium imaging, so that a final concentration of sucrose became 100 mM. A fluorescent image (excitation at 340 nm and 380 nm, fluorescence at 510 nm) in the field of a microscope after 30 seconds from addition of the sucrose solution was taken, and a cell response by sucrose was observed by displaying a ratio of 2-wavelength excitation fluorescence by a pseudocolor. Results are shown in Fig. 4.

In the same manner as in Example 6, stimulation was performed at room temperature by adding a HEPES buffer containing conc. p-methoxycinnamic aldehyde to a cell to be subjected to fluorescent calcium imaging, so that a final concentration of p-methoxycinnamic aldehyde became 5 v/v%, and the state of the cell was observed by a fluorescent image. Results are shown in Fig. 4.

In the same manner as in Example 6, stimulation was performed at room temperature by adding a HEPES buffer containing sucrose to a cell to be subjected to fluorescent calcium imaging, so that a final concentration of sucrose became 100 mM, and further adding a HEPES buffer containing conc. p-methoxycinnamic aldehyde, so that a final concentration of p-methoxycinnamic aldehyde became 5 v/v%, and the state of the cell was observed by a fluorescent image. Results are shown in Fig. 4.

From the obtained results, it could be visually confirmed that a physiological response by sucrose is increased by adding p-methoxycinnamic aldehyde, in a cultured cell strain which is allowed to functionally stably express both of a human sweet taste receptor (hT1 R2+hT1 R3) and a chimeric G protein (hG16gust44).

### (Example 9)

### Physiological response of sweet taste receptor-expressing cell to sucrose stimulation, when cyclotene is added

How a physiological response of a sweet taste receptor-expressing cell by sucrose changes by adding cyclotene was analyzed by fluorescent calcium imaging using a fluorescent microscope.

In the same manner as in Example 6, stimulation was performed at room temperature by adding a HEPES buffer containing sucrose to a cell to be subjected to fluorescent calcium imaging, so that a final concentration of sucrose became 100 mM. A fluorescent image (excitation at 340 nm and 380 nm, fluorescence at 510 nm) in the field of a microscope after 30 seconds from addition of the sucrose solution was taken, and a cell response by sucrose was observed by displaying a ratio of 2-wavelength excitation fluorescence by a pseudocolor. Results are shown in Fig. 4.

In the same manner as in Example 6, stimulation was performed at room temperature by adding a HEPES buffer containing cyclotene to a cell to be subjected to fluorescent calcium imaging, so that a final concentration of cyclotene became 0.5 mM, and the state of the cell was observed by a fluorescent image. Results are shown in Fig. 4.

In the same manner as in Example 6, stimulation was performed at room temperature by adding a HEPES buffer containing sucrose to a cell to be subjected to fluorescent calcium imaging, so that a final concentration of sucrose became 100 mM, and further adding a HEPES buffer containing cyclotene, so that a final concentration of cyclotene became 0.5 mM, and the state of the cell was observed by a fluorescent image. Results are shown in Fig. 4.

From the obtained results, it could be visually confirmed that a physiological response by sucrose is increased by adding cyclotene, in a cultured cell strain which is allowed to functionally stably express both of a human sweet taste receptor (hT1 R2+hT1R3) and a chimeric G protein (hG16gust44).

### (Examples 10)

### Physiological response of sweet taste receptor-expressing cell to aspartame stimulation, when neohesperidin dihydrochalcone is added

How a physiological response of a sweet taste receptor-expressing cell by aspartame changes by adding neohesperidin dihydrochalcone was analyzed by automated fluorescent imaging.
To a cell to be subjected to automated fluorescent imaging, which had been prepared in the same manner as in Example 2, was added a HEPES buffer containing aspartame, so that a final concentration of aspartame became a concentration described in Table 6, thereby, aspartame stimulation was performed at 27°C.
A fluorescent reaction (excitation at 485 nm, fluorescence at 525 nm) from immediately after addition of a HEPES buffer containing aspartame to after 100 seconds was measured by using FlexStation 3 (Molecular Devices), and a response of a sweet taste receptor-expressing cell to sweet taste stimulation by aspartame was quantitated by automated fluorescent imaging. Results are shown in Table 6 and Fig. 5.

In the same manner as that described above, stimulation was performed at 27°C by adding a HEPES buffer containing aspartame to a cell to be subjected to automated fluorescent imaging, which had been prepared in the same manner as in Example 2, so that a final concentration of aspartame became a concentration described in Table 7, and further adding a HEPES buffer containing conc. neohesperidin dihydrochalcone, so that a final concentration of neohesperidin dihydrochalcone became 5 v/v%, a fluorescent reaction (excitation at 485 nm, fluorescence at 525 nm) from immediately after addition of the solution to after 100 seconds was measured using FlexStation 3 (Molecular Devices), and a response of a sweet taste receptor-expressing cell, when neohesperidin dihydrochalcone was added to aspartame, was quantitated. Results are shown in Table 7 and Fig. 5. The vertical axis of Fig. 5 is a maximum (ΔF) of a change amount in a fluorescent intensity between immediately after stimulation and after 100 seconds from stimulation, that is, a response intensity of a cell, and the horizontal axis indicates an aspartame concentration (mM) as expressed by logarithm.

From the obtained results, it was recognized that a physiological response by aspartame is synergistically raised by adding neohesperidin dihydrochalcone, in a cultured cell strain which is allowed to functionally stably express both of a human sweet taste receptor (hT1R2+hT1R3) and a chimeric G protein (hG16gust44).

**[Table 6]**

| Aspartame (mM) | Change amount in fluorescent intensity (ΔF) |
|---|---|
| 0.167 | 6.568 |
| 0.333 | 12.578 |
| 0.667 | 33 |
| 2 | 72.005 |

**[Table 7]**

| Aspartame (mM) | NHDC (V/V%) | Change amount in fluorescent intensity (ΔF) |
|---|---|---|
| 0.167 | 5 | 30.148 |
| 0.333 | 5 | 40.039 |
| 0.667 | 5 | 61.367 |
| 2 | 5 | 101.285 |

### (Examples 10)

### Physiological response of sweet taste receptor-expressing cell to stevia stimulation, when neohesperidin dihydrochalcone is added

How a physiological response of a sweet taste receptor-expressing cell by stevia changes by adding neohesperidin dihydrochalcone was analyzed by automated fluorescent imaging.
To a cell to be subjected to automated fluorescent imaging, which had been prepared in the same manner as in Example 2, was added a HEPES buffer containing stevia, so that a final concentration of stevia became a concentration described in Table 8, thereby, stevia stimulation was performed at 27°C.
A fluorescent reaction (excitation at 485 nm, fluorescence at 525 nm) from immediately after addition of a HEPES buffer containing stevia to after 40 seconds was measured by using FlexStation 3 (Molecular Devices), and a response of a sweet taste receptor-expressing cell to sweet taste stimulation by stevia was quantitated by automated fluorescent imaging. Results are shown in Table 8 and Fig. 6.

In the same manner as that described above, stimulation was performed at 27°C by adding a HEPES buffer containing stevia to a cell to be subjected to automated fluorescent imaging, which had been prepared in the same manner as in Example 2, so that a final concentration of stevia became a concentration described in Table 9, and further adding a HEPES buffer containing conc. neohesperidin dihydrochalcone, so that a final concentration of neohesperidin dihydrochalcone became 5 v/v%, a fluorescent reaction (excitation at 485 nm, fluorescence at 525 nm) from immediately after addition of the solution to after 100 seconds was measured using FlexStation 3 (Molecular Devices), and a response of a sweet taste receptor-expressing cell, when neohesperidin dihydrochalcone was added to stevia, was quantitated. Results are shown in Table 9 and Fig. 6. The vertical axis of Fig. 6 is a maximum (ΔF) of a change amount in a fluorescent intensity between immediately after stimulation and after 100 seconds from stimulation, that is, a response intensity of a cell, and the horizontal axis indicates an stevia concentration (mg/ml) as expressed by logarithm.

From the obtained results, it was recognized that a physiological response by stevia is synergistically raised by adding neohesperidin dihydrochalcone, in a cultured cell strain which is allowed to functionally stably express both of a human sweet taste receptor (hT1R2+hT1R3) and a chimeric G protein (hG16gust44).

**[Table 8]**

| Stevia (mg/ml) | Change amount in fluorescent intensity (ΔF) |
|---|---|
| 0.167 | 31.881 |
| 0.333 | 80.909 |
| 0.667 | 112.358 |
| 2 | 124.02 |

**[Table 9]**

| Stevia (mg/ml) | NHDC (V/V%) | Change amount in fluorescent intensity (ΔF) |
|---|---|---|
| 0.167 | 5 | 68.407 |
| 0.333 | 5 | 111.239 |
| 0.667 | 5 | 135.572 |
| 2 | 5 | 130.109 |

### (Examples 10)

### Physiological response of sweet taste receptor-expressing cell to saccharin stimulation, when neohesperidin dihydrochalcone is added

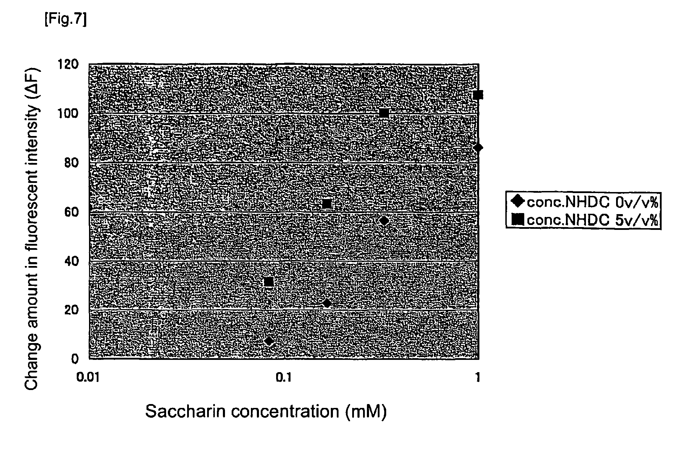

How a physiological response of a sweet taste receptor-expressing cell by saccharin changes by adding neohesperidin dihydrochalcone was analyzed by automated fluorescent imaging.
To a cell to be subjected to automated fluorescent imaging, which had been prepared in the same manner as in Example 2, was added a HEPES buffer containing saccharin, so that a final concentration of saccharin became a concentration described in Table 10, thereby, saccharin stimulation was performed at 27°C.
A fluorescent reaction (excitation at 485 nm, fluorescence at 525 nm) from immediately after addition of a HEPES buffer containing saccharin to after 100 seconds was measured by using FlexStation 3 (Molecular Devices), and a response of a sweet taste receptor-expressing cell to sweet taste stimulation by saccharin was quantitated by automated fluorescent imaging. Results are shown in Table 10 and Fig. 7.

In the same manner as that described above, stimulation was performed at 27°C by adding a HEPES buffer containing saccharin to a cell to be subjected to automated fluorescent imaging, which had been prepared in the same manner as in Example 2, so that a final concentration of saccharin became a concentration described in Table 11, and further adding a HEPES buffer containing conc. neohesperidin dihydrochalcone, so that a final concentration of neohesperidin dihydrochalcone became 5 v/v%, a fluorescent reaction (excitation at 485 nm, fluorescence at 525 nm) from immediately after addition of the solution to after 40 seconds was measured using FlexStation 3 (Molecular Devices), and a response of a sweet taste receptor-expressing cell, when neohesperidin dihydrochalcone was added to saccharin, was quantitated. Results are shown in Table 11 and Fig. 7. The vertical axis of Fig. 7 is a maximum (ΔF) of a change amount in a fluorescent intensity between immediately after stimulation and after 100 seconds from stimulation, that is, a response intensity of a cell, and the horizontal axis indicates an saccharin concentration (mM) as expressed by logarithm.

From the obtained results, it was recognized that a physiological response by saccharin is synergistically raised by adding neohesperidin dihydrochalcone, in a cultured cell strain which is allowed to functionally stably express both of a human sweet taste receptor (hT1 R2+hT1 R3) and a chimeric G protein (hG16gust44).

**[Table 10]**

| Saccharin (mM) | Change amount in fluorescent intensity (ΔF) |
|---|---|
| 0.167 | 7.609 |
| 0.333 | 22.936 |
| 0.667 | 56.59 |
| 2 | 86.194 |

**[Table 11]**

| Saccharin (mM) | NHDC (V/V%) | Change amount in fluorescent intensity (ΔF) |
|---|---|---|
| 0.167 | 5 | 31.699 |
| 0.333 | 5 | 63.454 |
| 0.667 | 5 | 100.311 |
| 2 | 5 | 107.555 |

### (Example 11)

### Physiological response of sweet taste receptor-expressing cell to sucrose stimulation, when ethyl vanillin or maltol is added

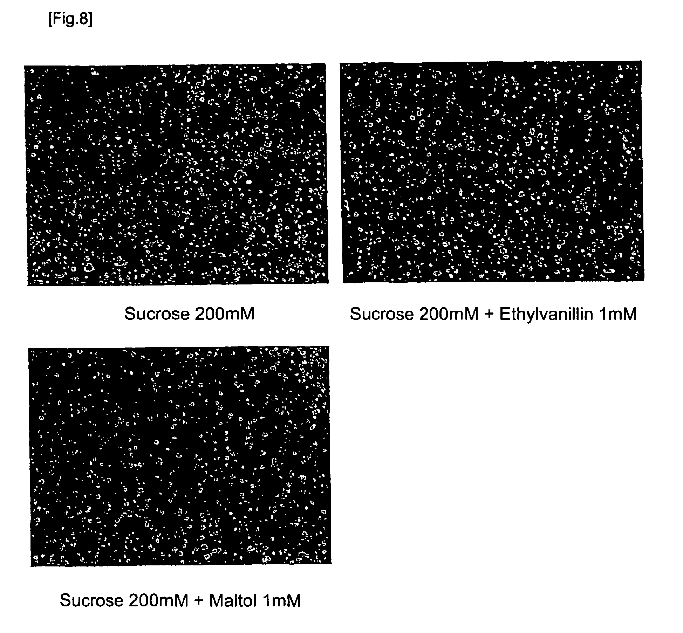

How a physiological response of a sweet taste receptor-expressing cell by sucrose changes by adding ethyl vanillin or maltol was analyzed by fluorescent calcium imaging using a fluorescent microscope.
In the same manner as in Example 2, sucrose stimulation was performed at room temperature by adding a HEPES buffer containing sucrose to a cell to be subjected to automated fluorescent imaging, so that a final concentration of sucrose became 200 mM.
A florescent image (excitation at 340 nm and 380 nm, fluorescence at 510 nm) in the field of a microscope after 30 seconds from addition of the sucrose solution was taken, and a cell response by sucrose was observed, by expressing a ratio of 2-wavelength excitation fluorescence by a pseudocolor. Results are shown in Fig. 8.

In the same manner as that described above, stimulation was performed at room temperature by adding a HEPES buffer containing sucrose to a cell to be subjected to fluorescent calcium imaging, so that a final concentration of sucrose became 200 mM, and further adding a HEPES buffer containing ethyl vanillin, so that a final concentration of ethyl vanillin became 1 mM, and the state of the cell was observed by a fluorescent image. Results are shown in Fig. 8.

In the same manner as that described above, stimulation was performed at room temperature by adding a HEPES buffer containing sucrose to a cell to be subjected to fluorescent calcium imaging, so that a final concentration of sucrose became 200 mM and, further, adding a HEPES buffer containing maltol, so that a final concentration of maltol became 1 mM, and the state of the cell was observed by a fluorescent image. Results are shown in Fig. 8.

From the obtained results, it could be visually confirmed that a physiological response by sucrose is suppressed by adding ethyl vanillin or maltol, in a cultured cell strain which is allowed to functionally stably express both of a human sweet taste receptor (hT1 R2+hT1 R3) and a chimeric G protein (hG16gust44).

### Industrial Applicability

According to the present invention, there can be provided a human sweet taste receptor-acting sweet taste regulating substance, whose sweet taste regulating effect through a human sweet taste receptor has been objectively confirmed, and advantageous effects such as improvement in a taste, saving of a sweetener, reduction in calorie, low caries etc. can be obtained by applying to wide foods and beverages.

## Claims

1. A human sweet taste receptor-acting sweet taste regulating substance to a sweet taste substance, which is identified by measurement of a physiological response by a sweet taste substance, using a cultured cell strain which is allowed to express hT1R2 and hT1R3, and a G protein α subunit, by transferring an expression construct obtained by inserting respective cDNAs encoding the hT1 R2 and the hT1 R3, and the G protein α subunit into the same plasmid, into a 293 cell in which an FRT (Flippase Recognition Target) site has been incorporated into one place in a genome DNA.

2. The human sweet taste receptor-acting sweet taste regulating substance according to claim 1, wherein the G protein α subunit is hG16gust44.

3. The human sweet taste receptor-acting sweet taste regulating substance according to claim 1 or 2, wherein the sweet taste substance is sucrose.

4. The human sweet taste receptor-acting sweet taste regulating substance according to any one of claims 1 to 3, wherein measurement of a physiological response to a sweet taste substance is measurement by calcium imaging.

5. The human sweet taste receptor-acting sweet taste regulating substance according to any one of claims 1 to 4, wherein the human sweet taste receptor-acting sweet taste regulating substance is a human sweet taste receptor-acting sweet taste enhancing substance.

6. A human sweet taste receptor-acting sweet taste enhancing agent comprising a human sweet taste receptor-acting sweet taste regulating substance according to claim 5.

7. A human sweet taste receptor-acting sweet taste enhancing agent to a sweet taste substance, comprising neohesperidin dihydrochalcone (NHDC), cyclamate, p-methoxycinnamic aldehyde or cyclotene.

8. The human sweet taste receptor-acting sweet taste regulating substance according to any one of claims 1 to 4, wherein the human sweet taste receptor-acting sweet taste regulating substance is a human sweet taste receptor-acting sweet taste suppressing substance.

9. A human sweet taste receptor-acting sweet taste suppressing agent comprising a human sweet taste receptor-acting sweet taste regulating substance according to claim 8.

10. A sweet taste receptor-acting sweet taste suppressing agent comprising lactisole, vanillin, ethylvanillin, creosol, or maltol.
